# EUROPÄISCHE PATENTSCHRIFT

(11) **EP 1 047 658 B1**
(45) Veröffentlichungstag und Bekanntmachung des Hinweises auf die Patenterteilung: **04.09.2002**
(21) Anmeldenummer: 98965767.1
(22) Anmeldetag: 04.12.1998
(51) Int. Cl.: C07C 67/26, C07C 69/28, C07C 69/52

(54) **VERFAHREN ZUR HERSTELLUNG VON ALKOXYLIERTEN DIMERFETTSÄUREN**
METHOD FOR PRODUCING ALKOXYLATED DIMER FATTY ACIDS
PROCEDE DE PRODUCTION D'ACIDES GRAS DIMERES ALCOCYLES

(30) Priorität: 13.12.1997 DE 19755559
(43) Veröffentlichungstag der Anmeldung: 02.11.2000
(73) Patentinhaber: Cognis Deutschland GmbH & Co. KG, 40589 Düsseldorf (DE)
(72) Erfinder: RATHS, Hans-Christian, D-40789 Monheim (DE); BONGARDT, Frank, D-40670 Meerbusch (DE); BEHLER, Ansgar, D-46240 Bottrop (DE); RÖDER, Jürgen, D-40589 Düsseldorf (DE)
(86) Internationale Anmeldenummer: EP9807907
(87) Internationale Veröffentlichungsnummer: WO99031040

(56) Entgegenhaltungen:
- EP-A- 0 178 913
- DE-A- 2 024 050
- BARTHOLOME ET AL.: "Ullmanns Encyklopädie der technischen Chemie Band 4" 1976 , VERLAG CHEMIE , WEINHEIM XP002099719 siehe Seite 540

## Beschreibung

Die Erfindung betrifft ein Verfahren zur Herstellung von Alkylenglykolestern von Dimerfettsäuren durch Alkoxylierung von Dimerfettsäuren in Gegenwart von Alkanolaminen.

Die Anlagerung von Alkylenoxiden an CH-acide Verbindungen wie beispielsweise Fettalkohole, Alkylphenole, Fettamine oder auch Fettsäuren zählt zu den großtechnisch etablierten Verfahren zur Herstellung von nichtionischen Tensiden. Üblicherweise werden diese Reaktionen in Gegenwart homogener basischer Katalysatoren wie beispielsweise Natriumhydroxid oder Natriummethylat durchgeführt. Die Alkoxylierung stellt jedoch eine wenig selektive Reaktion dar, so daß man in der Praxis findet, daß insbesondere bei niedrigen Alkoxylierungsverhältnissen das Maximum der resultierenden Homologenverteilung nicht mit dem durchschnittlichen Alkoxylierungsgrad übereinstimmt.

Man versucht diesem unerwünschten Effekt zu begegnen, indem man Katalysatoren einsetzt, die eine höhere Selektivität aufweisen und in Summe zu Alkoxylaten, speziell Ethoxylaten, mit engerer ("eingeengter") Homologenverteilung führen; diese Produkte werden in der Literatur häufig auch als "narrow-range ethoxylates" bezeichnet. Als homogene Katalysatoren kommen für diesen Zweck vorzugsweise Erdalkalisalze, wie beispielsweise Bariumphosphat oder Strontiumethercarboxylate in Frage. Auch heterogene Katalysatoren wie beispielsweise calcinierte Hydrotalcite eignen sich hierfür.

Bezüglich der Ethoxylierung von Fettsäuren sind die Bemühungen nach dem Stand der Technik indes nach wie vor nicht befriedigend. Insbesondere wenn es darum geht, niedrig ethoxylierte Fettsäuren, speziell Fettsäure + 1EO-Addukte, herzustellen, die als Vorstufen zur Synthese von Ethersulfat-Tensiden mit isethionatartiger Struktur interessant sind, werden nicht befriedigende Selektivitäten festgestellt. Neben einem unerwünschten Anteil höher ethoxylierter Homologer werden dabei insbesondere auch signifikante Mengen an Polyethylenglycol und Diestern gebildet. Auch das Verfahren gemäß dem US-Patent **US 3,884,946** (Henkel), das für diesen Zweck die Verwendung von Aminen als Katalysatoren empfiehlt, liefert die niedrig ethoxylierten Fettsäuren in Ausbeuten deutlich unter 90 % der Theorie. Bessere Ausbeuten können für niedrig ethoxylierte Fettsäuren erhalten werden, wenn gemäß der amerikanischen Patentanmeldung Serial No. **08/767.123** gearbeitet wird.

Nach diesem Verfahren wird in die Ethoxylierung in Gegenwart von Alkanolaminen als Katalysatoren durchgeführt und liefert deutlich höhere Ausbeuten.

Gemäß der europäischen Offenlegungsschrift **EP-A-178 913** können nicht nur geradkettige Fettsäuren, sondern auch verzweigte Neocarbonsäuren mit einem tertiären Kohlenstoffatom in Nachbarstellung zur Carboxylgruppe in Gegenwart von Aminen wie Diethanolamin und Triethanolamin mit erhöhter Selektivität alkoxyliert werden. Um nach diesem Verfahren gute Ausbeuten zu erhalten, sind jedoch relativ hohe Temperaturen im Bereich von 140 bis 185 °C notwendig.

Die DE-OS 20 24 050 beschreibt ein Verfahren zur Herstellung von Alkylenglycolestern von Carbonsäuren mit 4 bis 26 C-Atomen, wobei diese bei Temperaturen von vorzugsweise 50 bis 100° C in Gegenwart von AminKatalysatoren ethoxyliert werden.

Gemäß dem zitierten Stand der Technik wurde das Problem der Selektivität bei der Alkoxylierung nur für monomere Carbonsäuren untersucht. Bislang nicht betrachtet wurde jedoch das Problem der selektiven Alkoxylierung bei oligomeren Carbonsäuren, insbesondere bei Dimerfettsäuren. Es bestand demnach ein Bedürfnis auch für die Alkoxylierung von Dimerfettsäuren ein selektives Verfahren zur Verfügung zu stellen.

Demzufolge hat die Aufgabe der vorliegenden Erfindung darin bestanden, ein verbessertes homogenkatalytisches Verfahren zur Herstellung von Alkylenglykolestern von Dimerfettsäuren, speziell niedrig alkoxylierten Dimerfettsäuren, zur Verfügung zu stellen, das sich durch eine verbesserte Selektivität auszeichnet.

Überraschenderweise konnte die Aufgabe gelöst werden, indem Alkanolamine, speziell Triethanolamin, als Katalysator bei der Alkoxylierung der Dimerfettsäuren zugegen ist. Besonders geeignet ist dieses Verfahren, wenn es darum geht, niedrig alkoxylierte Dimerfettsäuren herzustellen.

Ein Gegenstand der vorliegenden Erfindung ist ein Verfahren zur Herstellung von Alkylenglykolestern von Dimerfettsäuren durch Anlagerung von Alkylenoxid an Dimerfettsäuren in Gegenwart von Alkanolaminen als Katalysatoren, wobei man Dimerfettsäuren einsetzt, die durch Oligomerisierung von technisch ungesättigten Fettsäuren mit 16 bis 18 Kohlenstoffatomen hergestellt werden und die Alkoxylierung selbst bei Temperaturen im Bereich von 110 bis 140° C bei autogenen Drücken im Bereich von 1 bis 5 bar durchgeführt werden.

Im Sinne der vorliegenden Erfindung werden die Begriffe "alkoxylierte Dimerfettsäuren" und "Alkylenglykolester von Dimerfettsäuren" synonym gebraucht,

### Dimerfettsäuren

Im Sinne der Erfindung sind unter Dimerfettsäuren technische Gemische zu verstehen, die durch Oligomerisierung von ungesättigten Fettsäuren oder deren Methylester erhalten werden.

Die Oligomerisierung von ungesättigten Fettsäuren stellt eine bekannte elektrocyclische Reaktion dar, über die in Übersichtsartikeln beispielsweise von A. Behr in **Fat Sci. Technol. 93, 340 (1991),** G.Spiteller in **Fat Sci. Technol. 94, 41 (1992)** oder P. Daute et al. in **Fat Sci. Technol. 95, 91 (1993)** berichtet wird. Bei der Oligomerisierung treten durchschnittlich zwei bis drei Fettsäuren zusammen und bilden Dimere bzw. Trimere, die überwiegend cycloaliphatische Strukturen aufweisen. Neben der Fraktion der Dimeren und Trimeren wird eine sogenannte Monomerfraktion erhalten, in der sich nicht umgesetzte Ausgangsstoffe und verzweigte Monomere befinden, die im Verlauf der Reaktion durch Isomerisierung entstanden sind. Daneben gibt es selbstverständlich auch eine Fraktion höherer Oligomeren, die jedoch in der Regel nicht von größerer Bedeutung ist. Die Oligomerisierung kann thermisch oder in Gegenwart von Edelmetallkatalysatoren durchgeführt werden. Vorzugsweise erfolgt die Reaktion in Gegenwart von Tonerden wie beispielsweise Montmorillonit [vgl. **Fette, Seifen, Anstrichmitt. 72, 667 (1970)**]. Die Regelung des Gehaltes an Dimeren und Trimeren bzw. der Umfang der Monomerfraktion kann durch die Reaktionsbedingungen gesteuert werden. Technische Gemische können schließlich auch destillativ aufgereinigt werden.

Als Ausgangsstoffe für die Oligomerisierung kommen technische ungesättigte Fettsäuren mit 12 bis 22, vorzugsweise 16 bis 18, Kohlenstoffatomen in Betracht. Typische Beispiele sind Palmoleinsäure, Ölsäure, Elaidylsäure, Petroselinylsäure, Linolsäure, Linolensäure, Konjuenfettsäure, Elaeostearinsäure, Ricinolsäure, Gadoleinsäure, Erucasäure sowie deren technische Gemische mit gesättigten Fettsäuren. Typische Beispiele für geeignete technische Gemische sind ungehärtete Spaltfettsäuren natürlicher Triglyceride mit Iodzahlen im Bereich von 40 bis 140, wie etwa Palmfettsäure, Talgfettsäure, Rübölfettsäurem, Sonnenblumenfettsäure und dergleichen. Bevorzugt sind Spaltfettsäuren mit einem hohen Gehalt an Ölsäure.

Neben den Fettsäuren können auch deren Ester, vorzugsweise Methylester dimerisiert werden. Es ist gleichfalls möglich, die Säure zu oligomerisieren und vor der Hydrierung in die Methylester zu überführen. Die Überführung der Estergruppe in die Säuregruppe gelingt in an sich bekannter Weise.

Dimerfettsäuren, die im Sinne der Erfindung besonders bevorzugt sind, werden durch Oligomerisierung von technischer Ölsäure erhalten und weisen vorzugsweise einen Dimergehalt von 50 bis 99 Gew.-% sowie einen Polymergehalt (inklusive Trimergehalt) von 1 bis 50 Gew.-% auf. Der Gehalt an Monomeren kann 1 bis 15 Gew.-% betragen und falls erforderlich durch Destillation erniedrigt werden. Insbesondere bevorzugt werden Dimerfettsäuren, die durch Oligomerisierung von technischer Ölsäure erhalten werden und einen Dimergehalt von 70 bis 85 Gew.%, einen Polymergehalt von 10 bis 20 Gew.% und einen Monomergehalt von 5 bis 15 Gew.% aufweisen. Die Gew.% sind dabei auf die Gesamtmenge an Dimerfettsäure bezogen.

### Alkanolamine

Typische Beispiele für Alkanolamine, die als homogene basische Katalysatoren in Betracht kommen, sind Monoethanolamin, Diethanolamin und vorzugsweise Triethanolamin. Weiterhin kommt auch Amine auf Basis von Diolen als Katalysatoren in Frage, vorzugsweise Diglykolamin. Üblicherweise werden die Alkanolamine in Mengen von 0,05 bis 5, vorzugsweise 0,1 bis 1,5 Gew.-% - bezogen auf die Dimerfettsäuren - eingesetzt.

### Alkoxylierung

Die Alkoxylierung kann in an sich bekannter Weise durchgeführt werden und soll im folgenden exemplarisch an der Ethoxylierung beschrieben werden.

Üblicherweise legt man die Dimerfettsäure und den Katalysator in einem Rührautoklaven vor, den man vor der Reaktion durch abwechselndes Evakuieren, vorzugsweise bei Temperaturen im Bereich von 80 bis 120°C, und Stickstoffspülen von Wasserspuren befreit. Anschließend wird die Dimerfettsäure mit dem Ethylenoxid, welches man nach dem Aufheizen portionsweise über einen Heber in den Druckbehälter eindosieren kann, umgesetzt.

Vorzugsweise liegt das molare Umsetzungsverhältnis von Dimerfettsäure zu Ethylenoxid im Bereich von 1 : 0,5 bis 1 : 6,0, vorzugsweise 1: 1 bis 1 : 3,0. Besondere Vorteile hinsichtlich der Selektivität zeigt das Verfahren, wenn pro Mol Dimerfettsäure etwa zwei Mol Ethylenoxid umgesetzt werden (molares Einsatzverhältnis 1:2).

Erfindungsgemäß wird die Ethoxylierung bei 110 bis 140°C und insbesondere bei 115 bis 125 °C durchgeführt. Bei der Ethoxylierung empfehlen sich autogene Drücke im Bereich von 1 bis 5, vorzugsweise im Bereich von 2 bis 4 bar. Nach Reaktionsende empfiehlt es sich, zur Vervollständigung des Umsatzes eine gewisse Zeit bei der Reaktionstemperatur und den autogenen Drücken nachzurühren (15 bis 90min). Anschließend wird der Autoklav abgekühlt, entspannt und das Produkt, falls dies gewünscht wird, mit Säuren wie z.B. Milchsäure oder Phosphorsäure versetzt, um den basischen Katalysator zu neutralisieren.

Sinngemäß gelten die obigen Ausführungen zur reinen Ethoxylierung auch für die reine Propoxylierung und für die gemischte Ethoxylierungs- und Propoxylierungsreaktion, wobei bei letzterem je nach Wunsch entweder eine Mischung von Ethylenoxid und Propylenoxid mit den Dimerfettsäuren umgesetzt werden kann oder erst Ethylenoxid und dann Propylenoxid oder umgekehrt. Prinzipiell ist das erfindungsgemäße Verfahren auch für die Alkoxylierung mit Butylenoxid geeignet, aber bevorzugt wird die Alkoxylierung mit Ethylenoxid und/oder Propylenoxid, insbesondere nur mit Ethylenoxid, durchgeführt.

Nach dem erfindungsgemäßen Verfahren werden Alkylenglykolester von Dimerfettsäuren in guten Ausbeuten erhalten, wobei es insbesondere gelungen ist, den Anteil von Dieestem, als Reaktionsprodukte, die durch Umsetzung zweier Dimerfettsäuren untereinander entstehen, der bei den hochmolekularen Dimerfettsäuren besonders störend ist, zu reduzieren. So weisen die nach dem erfindungsgemäßen Verfahren hergestellten Produkte vorzugsweise einen Monoestergehalt über 85 Gew.%, insbesondere über 90 Gew.% und einen Diestergehalt unter 7 Gew.%, vorzugsweise unter 5 Gew.% auf - bezogen auf Verfahrensprodukt-. Der zu 100 Gew.% fehlende Rest stellt nicht umgesetzte Restsäure dar.

Die nach dem erfindungsgemäßen Verfahren als Hauptprodukt hergestellten Alkylenglykolester können vereinfacht durch die Formel (I) beschrieben werden,

**H-(OR)**_{**n**}**OOC - R**^{**1**} **- COO(RO)**_{**n**}**-H** (I)

in der R¹ für einen Kohlenwasserstoffrest einer Dimerfettsäure mit 24 bis 44 C-Atomen und R jeweils unabhängig voneinander für einen Alkylrest mit 2 oder 3 C-Atomen, insbesondere für CH₂CH₂, CHCH₃CH₂ und/oder CH₂CHCH₃ und n jeweils für etwa die gleiche Zahl im Bereich von 0,25 bis 3,0 steht,
wiedergegeben werden. Diese Formel ist insofern vereinfacht, als zugrundegelegt wurde, daß die eingesetzten Dimerfettsäuren ausschließlich dimere Carbonsäuren sind. Wie oben beschrieben enthalten die Dimerfettsäuren als technische Gemische jedoch auch anteilsweise monomere sowie trimere und weitere polymere Carbonsäuren. Da die im Sinne der Erfindung bevorzugten Dimerfettsäuren als Hauptbestandteil vorliegen, steht die Formel (I) für Verbindungen, die als Hauptbestandteil in den Produkten enthalten sind, die nach dem erfindungsgemäßen Verfahren anfallen.

### Beispiele

### Beispiel 1:

In einem Autoklaven wurden 865,5 g (1,5 Mol) Dimerfettsäure (hergestellt durch Oligomerisierung von technischer Ölsäure; Monomergehalt 9 Gew.%, Dimergehalt 77 Gew.%, Polymergehalt 14 Gew.%, Säurezahl 189-197; Verseifungszahl 195, Molekulargewicht etwa 577g/mol) vorgelegt und mit 5 g Triethanolamin (entsprechend 0,58 Gew.-% bezogen auf Dimerfettsäure) versetzt. Der Autoklav wurde dreimal abwechselnd 30 Minuten bei 80°C und 30 mbar evakuiert und mit Stickstoff beaufschlagt um Spuren von Wasser, die zur Bildung von Polyethylenglycol führen könnten, zu entfernen. Nachdem die Reaktionsmischung ein letztesmal mit Stickstoff beaufschlagt worden war, wurde der Autoklav verschlossen auf 120°C erhitzt und portionsweise bei einem Maximaldruck von 5 bar mit 132,3 g (3 Mol) Ethylenoxid beaufschlagt. Nach Abschluß der Reaktion, erkenntlich dadurch, daß der Druck wieder bis auf einen Wert von 2 bar abfiel und dann konstant blieb, wurde 60 min nachgerührt bei 120 °C und 5 bar und der Reaktionsansatz anschließend abgekühlt und entspannt. Der basische Katalysator verblieb im Endprodukt.

Es wurde ein Produkt erhalten, welches einen Dimerestergehalt mit 1 Mol Ethylenoxid pro Carboxylgruppe von 90,7 Gew.%, einen Dimerestergehalt mit mehr als 1 Mol Ethylenoxid pro Carboxylgruppe von 2,8 Gew.%, einen Anteil an Diestern von 3,2 Gew.% und einen Restsäuregehalt von 3,1 Gew.% aufwies.

Die Zusammensetzung des Produktes zeigt, daß zum einen das erfindungsgemäße Verfahren in hohen Ausbeuten Dimerfettsäureester erzeugt, die keine unveresterten Carboxylgruppen mehr enthalten und zum anderen, daß das erfindungsgemäße Verfahren äußerst selektiv ist, da die überwiegende Menge der Dimerester solche Verbindungen sind, die pro Mol Carboxylgruppe der Dimerfettsäure nur 1 Mol Ethylenoxid - oder entsprechend den eingesetzten Mengen an Ethylenoxid gewünschte Mengen - aufweisen.

### Beispiel 2:

Es wurde unter den gleichen Bedingungen wie im Beispiel 1 beschrieben ein Dimerfettsäureester hergestellt. Zum Unterschied wurde als Katalysator Diglykolamin eingesetzt. Es wurden 1125 g Dimerfettsäure (hergestellt durch Oligomerisierung von Linolsäure mit Monomergehalt 9 Gew.-%, Dimergehalt 77 Gew.-% und Polymergehalt 14 Gew.-% sowie Säurezahl 124) in Gegenwart von 6,5 g (0,5 Gew.-% bezogen auf Dimerfettsäure) Diglykolamin und 176 g Ethylenoxid zur Reaktion gebracht. Das Endprodukt wies einen Dimerestergehalt von mit 1 Mol Ethylenoxid pro Carboxylgruppe ethoxyliertem Produkt von 92 Gew.-% auf. Die Säurezahl betrug 4,6. Die OH-Zahl wurde mit 160 gemessen.

## Patentansprüche

1. Verfahren zur Herstellung von Alkylenglykolmonoestern von Dimerfettsäuren durch Anlagerung von Alkylenoxid an Dimerfettsäuren in Gegenwart von Alkanolaminen als Katalysatoren, **dadurch gekennzeichnet, daß** man Dimerfettsäuren einsetzt, die durch Oligomerisierung von technisch ungesättigten Fettsäuren mit 16 bis 18 Kohlenstoffatomen hergestellt werden und die Alkoxylierung bei Temperaturen im Bereich von 110 bis 140 °C bei autogenen Drücken im Bereich von 1 bis 5 bar durchgeführt wird.

2. Verfahren nach Anspruch 1, **dadurch gekennzeichnet, daß** man Dimerfettsäuren einsetzt, die durch Oligomerisierung von technischer Ölsäure hergestellt worden sind.

3. Verfahren nach den Ansprüchen 1 und 2, **dadurch gekennzeichnet, daß** man die Dimerfettsäuren mit Ethylenoxid umsetzt.

4. Verfahren nach den Ansprüchen 1 bis 3, **dadurch gekennzeichnet, daß** man die Dimerfettsäuren mit Ethylenoxid im molaren Verhältnis von 1: 0,5 bis 1: 6,0, insbesondere im Verhältnis von 1 : 1 bis 1 : 3, umsetzt.

5. Verfahren nach den Ansprüchen 1 bis 4, **dadurch gekennzeichnet, daß** man als Alkanolamin Triethanolamin einsetzt.

6. Verfahren nach den Ansprüchen 1 bis 5, **dadurch gekennzeichnet, daß** man die Alkanolamine in Mengen von 0,05 bis 5 Gew.-%- bezogen auf die Dimerfettsäuren - einsetzt.

## Claims

1. A process for the production of alkylene glycol monoesters of dimer fatty acids by addition of alkylene oxide onto dimer fatty acids in the presence of alkanolamines as catalysts, **characterized in that** dimer fatty acids obtained by oligomerization of technically unsaturated C₁₆₋₁₈ fatty acids are used and the alkoxylation is carried out at temperatures of 110 to 140°C under autogenous pressures of 1 to 5 bar.

2. A process as claimed in claim 1, **characterized in that** dimer fatty acids obtained by oligomerization of technical oleic acid are used.

3. A process as claimed in claims 1 and 2, **characterized in that** the dimer fatty acids are reacted with ethylene oxide.

4. A process as claimed in claims 1 to 3, **characterized in that** the dimer fatty acids are reacted with ethylene oxide in a molar ratio of 1:0.5 to 1:6.0 and more particularly in a ratio of 1:1 to 1:3.

5. A process as claimed in claims 1 to 4, **characterized in that** triethanolamine is used as the alkanolamine.

6. A process as claimed in claims 1 to 5, **characterized in that** the alkanolamines are used in quantities of 0.05 to 5% by weight, based on the dimer fatty acids.

## Revendications

1. Procédé de production de monoesters d'alkylène glycol d'acides gras dimères par fixation d'oxyde d'alkylène sur des acides gras dimères en présence d'alcanolamines comme catalyseurs,
**caractérisé en ce qu'**
on utilise des acides gras dimères que l'on produit par oligomérisation d'acides gras techniquement insaturés avec de 16 à 18 atomes de carbone et **en ce qu'**on réalise l'alcoxylation à des températures allant de 110 à 140°C à des pressions autogènes allant de 1 à 5 bars.

2. Procédé selon la revendication 1.
**caractérisé en ce qu'**
on utilise des acides gras dimères qui sont produits par oligomérisation d'acide oléique industriel.

3. Procédé selon les revendications 1 et 2,
**caractérisé en ce qu'**
on fait réagir des acides gras dimères avec de l'oxyde d'éthylène.

4. Procédé selon les revendications 1 à 3,
**caractérisé en ce qu'**
on fait réagir les acides gras dimères avec de l'oxyde d'éthylène dans un rapport molaire de 1:0,5 à 1:6,0, en particulier dans un rapport de 1:1 à 1:3.

5. Procédé selon les revendications 1 à 4,
**caractérisés en ce qu'**
on utilise comme alcanolamine la triéthanolamine.

6. Procédé selon les revendications 1 à 5,
**caractérisé en ce qu'**
on utilise les alcanolamines à des quantités de 0,05 à 5 % en poids - par rapport aux acides gras dimères.
